(19)

Europäisches
Patentamt
European
Patent Office
Office européen
des brevets

(11)     **EP 2 767 311 B1**

(12)     **EUROPEAN PATENT SPECIFICATION**

(45) Date of publication and mention
of the grant of the patent:
**10.01.2018  Bulletin 2018/02**

(51) Int Cl.:
*A61Q 9/04* (2006.01)      *A61K 8/81* (2006.01)
*A61K 8/02* (2006.01)

(21) Application number: **13155272.1**

(22) Date of filing: **14.02.2013**

(54) **Stable personal care article**

Stabiler Körperpflegeartikel

Article d'hygiène personnelle stable

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**20.08.2014  Bulletin 2014/34**

(73) Proprietor: **The Procter & Gamble Company
Cincinnati, OH 45202 (US)**

(72) Inventors:
• **Smith, Paul  James
Whitton, Middlesex TW2 6EB (GB)**
• **Fletcher, Jamie, Anthony
Luston, Hertfordshire HR6 OEB (GB)**

(74) Representative: **Kohol, Sonia
Technical Centres Limited
Procter & Gamble Patent Department
Rusham Park
Whitehall Lane
Egham, Surrey TW20 9NW (GB)**

(56) References cited:
**EP-A1- 2 356 967          WO-A1-2008/102125
WO-A1-2011/103222      WO-A2-02/11687
JP-A- 2001 054 422      JP-A- 2005 145 895
US-A1- 2002 146 380      US-A1- 2007 134 304**

• **DATABASE GNPD [Online] MINTEL; October
2012 (2012-10), "Shower, Scrub & Soften Deluxe
Set", XP002717198, Database accession no.
1962563**

**Description**

FIELD OF THE INVENTION

The present invention relates to personal care articles comprising a personal care composition disposed on a substrate, wherein the personal care composition comprises particles.

BACKGROUND OF THE INVENTION

Personal care compositions generally and depilatory compositions used to remove unwanted hair by chemical activity are known. Such compositions may comprise reducing agents to degrade keratin in the hair and thus weaken the hair strands. These compositions may take the form of creams, lotions and the like which may be applied to the body in a variety of ways, such as with a spatula. The spatula or another suitable implement may then used to scrape off the weakened hair strands and complete the depilation process, if a depilatory product has been used. This can be a messy and awkward procedure for the user of the personal care composition and provides no means of occluding the composition to prevent it from drying out. By disposing the personal care composition on a substrate one may overcome or mitigate such disadvantages. Substrate-based depilatory products are known from JP63073910A, US2006002878, JP6135826A, JP11012123A, JP62230711A and WO 02/11687 A2. While addressing some of the handling problems of creams and lotions by removing the need for an application implement, known substrate-based personal care compositions, especially oil-in-water emulsions, do not address the problems of maintaining the desired dose after manufacturing, storage and transport, or in use, nor the resultant risks of irritation or ineffective action that may be associated with uncontrolled dose. Specifically, applicants have found that substrate-based personal care compositions, particularly oil-in-water emulsions and polymeric gels, lead to the personal care composition displacing across the surface of a substrate, especially after manufacturing, storage and transport, but also during use, therefore leading to an undesired quantity of personal care composition being provided to different areas of the skin upon application of the composition. This uneven dose may lead to irritation in some areas and insufficient activity or benefit delivery in others. Simultaneously, this may also prevent conformability of the personal care composition to the surface of the body, thus inhibiting availability of active ingredients to the area for which cosmetic treatment is desired and further undermining the effectiveness of the composition. Consequently, there exists a need for an effective substrate-based personal care article that facilitates the desired dose of active ingredients being maintained after manufacturing, storage or transport and during use.

SUMMARY OF THE INVENTION

According to a first aspect of the invention, the applicants have surprisingly found that a personal care article as defined in claim 1 meets the aforementioned need by providing a robust personal care article that achieves a controlled dose of active ingredients after manufacturing, transport or storage and also in use.

According to a second aspect of the invention, a cosmetic method of treating the hair and or skin, preferably of removing hair from the skin is provided, comprising the steps of: applying a personal care article according to the first aspect of the invention to a surface of skin, preferably human skin, leaving said personal care article in contact with the skin for a period of greater than 1 minute, preferably from 2 to 10 minutes, more preferably from 2 to 8 minutes, removing said personal care article from the surface of the skin, and preferably rubbing, scraping, rinsing or wiping the surface of the skin in the area to which the personal care article was applied, as defined in claim 14.

According to a third aspect of the invention, a personal care kit, preferably a depilatory kit is also provided, comprising: a personal care article according to the first aspect of the invention, optionally at least one of a pre-treatment personal care composition, a post-treatment personal care composition and/or a tool to assist removal of hair and/or personal care composition after use, and packaging for said depilatory kit, as defined in claim 15.

DETAILED DESCRIPTION OF THE INVENTION

As used herein, the term "buffering base" refers to a base capable of opposing pH changes by means of chemical or physical (solubility) processes and thereby limiting the pH to less than or equal to 13.

As used herein, the term "water impermeable" includes materials or objects through which water in its liquid state does not pass.

As used herein the term "substantially free of volatile material or fragrance" refers to particles having less than 0.01%, preferably less than 0.005%, more preferably less than 0.0001% by weight of volatile and or fragrance material of said particles.

As used herein the term "particles" exclude particles comprising partially soluble or soluble materials wherein the solubility of said partially soluble or soluble materials at 25°C is at least 0.1 g/dm$^3$, preferably at least 0.5 g/dm$^3$,

more preferably at least 0.8 g/dm$^3$, even more preferably at least 1.0 g/dm$^3$ and even more preferably still at least 1.6 g/dm$^3$ in water.

As used herein, the term "particles percentage of the mean composition thickness2 is calculated by dividing the median particle diameter D(v,0.5), as measured on a Malvern Instruments Mastersizer 2000 with the Scirocco dry dispersion accessory, by the mean composition thickness and multiplying by 100.

As used herein "volatile" refers to materials with a boiling point at standard pressure below 200°C, preferably below 150°C, more preferably below 100°C, even more preferably below 75°C, even more preferably still below 50°C.

As used herein, the term mean composition thickness refers to the mean thickness of the personal care composition disposed on at least a portion of the first surface of said substrate.

As used herein, the term "sodium silicate" refers to sodium silicate and any other silicate comprising sodium. The same definition applies correspondingly to any other silicate, for example "potassium silicate" refers to potassium silicate and any other silicate comprising potassium, "ammonium silicate" to ammonium silicate and any other silicate comprising ammonium and "manganese silicate" to manganese silicate and any other silicate comprising manganese.

As used herein, all percentages are by weight of the personal care composition, unless otherwise specified.

Personal care articles according to the present invention comprise a personal care composition, preferably a depilatory composition, in contact with at least a portion of the first surface of a substrate, forming a coated region on the surface of the substrate. The personal care composition may be disposed on at least a portion of a first surface of the substrate, that surface being an active surface of the substrate. The personal care composition should be suitable for being placed in contact with a user's skin (and hair) and may be of any sort suitable for applying a cosmetic benefit to the user. Such cosmetic benefits include moisturizing the skin or hair, anti aging benefits such as fine line and wrinkle reduction, removal of unwanted hair, skin whitening, skin pigmentation, hair colouration, hair bleaching, shampoos, conditioning, hair growth, hair retardation, styling, deodorants, antiperspirants, personal cleansing and colour cosmetics. In a preferred embodiment, the personal care composition (or depilatory composition) is aqueous. The personal care composition comprises particles to improve the robustness of the personal care article towards damage incurred during manufacturing, packaging, storage and also during User handling and usage. The presence of the particles prevents the personal care composition from being excessively displaced. This would otherwise result in messiness and an uncontrolled dose of personal care composition being placed in contact with the User's skin (and hair) and ultimately lead to a less than desirable effectiveness of the personal care composition

The particles in the personal care composition may have a concentration of from 1% to 30.0% by weight, preferably from 1.5% to 15.0% by weight and more preferably from 2.5% to 10.0% by weight. The particles concentration enables sufficient dispersion throughout the personal care composition without limiting the composition levels of other ingredients necessary to achieve desirable effectiveness. Furthermore, if the concentration of the particles is too high, the feel of the personal care article with the User's skin can be unacceptable and the dose uncontrolled. The particles are preferably distributed homogenously throughout the personal care composition disposed on a portion of the first surface of the substrate.

The particles in the personal care composition may be present in an amount of from 0.15 g/cm$^2$ to 0.00001 g/cm$^2$, more preferably from 0.075 g/cm$^2$ to 0.00005 g/cm$^2$, even more preferably from 0.030 g/cm$^2$ to 0.0001 g/cm$^2$ and even more preferably still from 0.018 g/cm$^2$ to 0.00015 g/cm$^2$, wherein the unit area refers to the portion of coated region of the first surface of the substrate and not including any uncoated surface of the substrate. Additionally, the area used to calculate the amount of the personal care composition disposed upon the substrate is calculated ignoring any surface texturing or micro-structuring. The personal care composition disposed on at least a portion of the first surface of said substrate has a mean thickness of from 0.01mm to 5.00mm, preferably from 0.05mm to 2.50mm, even more preferably from 0.10mm to 1.00mm and even more preferably still from 0.15mm to 0.60mm. The mean composition thickness provides a sufficient dose to achieve the desired effectiveness, but not too high as to result in undesired effects such as irritation, messiness and difficulties in handling and conformability of the personal care article to the skin.

Advantageously, the particle percentage of mean composition thickness, calculated by dividing the median particle diameter D(v,0.5), as measured on a Malvern Instruments Mastersizer 2000 with the Scirocco dry dispersion accessory, by the mean composition thickness and multiplying by 100, is preferably from 10% to 100%, more preferably from 15% to 80%, even more preferably from 20% to 50% and even more preferably still from 25% to 40%. If the median particle diameter D(v,0.5) is less than 10% of the mean composition thickness it may be insufficient to reduce the damage that may result during manufacturing, packaging, storage and in use. If the median particle diameter D(v,0.5) is greater than the mean composition thickness, it may lead to an uncontrolled dose as well as an undesirable appearance and feel to the User and challenges in the manufacture of the personal care article. The particles may be dispersed within the personal care composition such that they do not extend beyond the thickness of the composition on the portion of the first surface of the substrate.

The particles are preferably solid. As used herein the term solid prefers to particles which are in a solid state of matter as distinct from liquids or gases. The particles may be solid throughout or have a hollow core comprising a gaseous material. The particles are preferably inert in the personal care composition and may be comprised of any natural or synthetic inert material including, but not limited to pulverized walnut husks, pulverized dried apricot seed, pulverized dried peach seed, powdered ceramics, glass, polymers (including polyolefins, such as polyethylene and polypropylene, polycarbonate; polyurethane; cellulose acetate, ethylene vinyl alcohol, thermo plastic elastomers, nylon), natural rubber, latex, silicones, waxes, talc, tricalcium phosphate, clays, zeolite and blends thereof. Preferably, the particles are comprised of polymers, and even more preferably polyolefins and even more preferably still polyethylene, polypropylene and mixtures thereof. Preferably the particles exhibit a hardness so that they do not deform under compression and are not too abrasive to the skin during the application, which may lead to irritation. Hardness is typically measured using the Moh's scale. The Moh's scale of hardness is a measure of the ability of one material to starch another. The scale ranges in order of increasing relative hardness from 1 (soft) to 10 (hardest). Preferably, the particles have a hardness according to the Moh's scale (i.e. soft) of less than 5, more preferably less than 3 and even more preferably less than 2, to avoid being harsh and irritating when in contact with the skin. Advantageously, the particles should not deform under compression applied to the personal care article during manufacturing, storage or handling by the User. Preferably, the particles should not disintegrate, undergo sedimentation, dissolve or chemically react in the personal care composition.

The particles may be of any shape including substantially regular, irregular and mixtures thereof such as spherical, elliptical, tetrahedron, octahedron, cuboid, dodecahedron (pentagonal faces), icosahedron (triangular faces) shaped. Preferably, the particles have a substantially irregular shape which may or may not be identical or similar. Without being bound by theory, it is believed that irregular particles prevent excessive movement of the particles in the personal care composition, which may result in an undesired accumulation in some regions and a decrease in other regions that may reduce their effectiveness.

The particles may have the same colour as the personal care composition or have a distinct colour to the personal care composition. The colour of the particles may advantageously change during the application due for example to changes in temperature, oxidation or mechanical compression. The colour of the particles may be selected to provide a desirable appearance of the personal care article or used to communicate instructions, such as indicating when the treatment has been completed.

Optionally, the particles may be coated or surface modified to achieve desired surface properties such as surface free energy, texture or encapsulation of another material. It may be advantageous for the particles to have a hydrophilic surface modification to enable effective dispersion in aqueous personal care composition. Surface modification may be achieved by any method known to those skilled in the art, such as acid treatment, plasma, corona treatment, particle spraying techniques, encapsulation technology, immersion, grinding and roughening.

The concentration of water in the personal care composition is at least 40%, more preferably from 50% to 98%, even more preferably from 60% to 95% and even more preferably still from 70% to 90%, by weight of the personal care composition. This high water level helps to improve the overall skin mildness of the personal care composition by being dilute, and to keep the system more robust to pH changes, which may result in skin irritation.

The rheological properties of the personal care composition may lead to further improvements and performance in use. In particular, the yield point describes the resistance of the personal care composition to deformation under environmental stress. If the yield point is too high, then the personal care composition may not deform sufficiently when applied to the surface of the body. In the case of a depilatory composition, this may lead to hair fibres being unable to enter the personal care composition effectively upon application, resulting in less desirable depilatory effectiveness. If the yield point is too low, however, then the personal care composition may flow readily and is not cleanly removed from the skin upon removal of the personal care article, thus requiring the inconvenience of additional wiping and risking irritation to the user. Accordingly, the personal care composition may have a yield point from 50 Pa to 2000 Pa, preferably from 65 Pa to 1200 Pa, more preferably from 80 Pa to 750 Pa and even more preferably from 100 Pa to 500 Pa when measured via a stress controlled amplitude sweep; at a frequency of 1Hz and a temperature of 25°C. The yield point described is defined as the 5% decrease in magnitude of the elastic modulus G' plateau value as measured on a TA1000 Rheometer, available from TA Instruments of New Castle, Delaware, USA. Once desired rheological properties have been specified, such as by the present invention, these properties may be altered in the personal care composition by changing the concentration or identity of the thickening system and the water content of the personal care composition.

Advantageously, the personal care composition may display an elastic modulus G' which exceeds its viscous modulus G" at all frequencies below 60 rad/s, preferably below 20 rad/s, more preferably below 10 rad/s and even more preferably below 1 rad/s; when measured via a strain controlled frequency sweep; at a strain of 1% and a temperature of 25°C. The elastic modulus of the personal care composition exceeds its viscous modulus at a low frequency of applied stress. This indicates that the personal care composition is behaving in a -like manner at rest and is of particular benefit when the personal care composition is interposed between two substrates, for example a substrate and a protective release layer.

In another preferred embodiment, the personal care composition displays a high degree of shear thinning behavior. Accordingly, at a low shear rate of 0.1 s$^{-1}$, the dynamic viscosity of the personal care composition is preferably 1000 Pa.s to 10000 Pa.s, whereas at a high shear rate of 1000 s$^{-1}$, the dynamic viscosity of the personal care composition is preferably 0.1 Pa.s to 1 Pa.s, measured at a temperature of 25°C.

To further reduce displacement of the personal care composition during storage or transit, the personal care composition may be disposed upon said substrate in an amount per unit area of from 0.500 g/cm$^2$ to 0.001 g/cm$^2$, more preferably from 0.250 g/cm$^2$ to 0.005 g/cm$^2$, even more preferably from 0.100 g/cm$^2$ to 0.010 g/cm$^2$ and even more preferably still from 0.060 g/cm$^2$ to 0.015 g/cm$^2$, wherein the unit area refers to the portion of coated region of the first surface of the substrate and not including any uncoated surface of the substrate. Additionally, the area used to calculate the amount of personal care composition disposed upon the substrate is calculated ignoring any surface texturing or micro-structuring.

Personal care articles of the present invention comprise a substrate to facilitate the application of the personal care composition to the skin and/or hair with a controlled dose of active ingredients and to prevent a messy usage experience. The substrate may be water permeable or water impermeable. The substrate may comprise any suitable material such as fibrous materials, papers, fabrics, non-wovens, plastics, amorphous solids, crystalline solids, metal based substrates such as foils, rubbers, latex, thermoplastic elastomers, cellular foams (open and closed cell), composites, laminates and mixtures thereof. Preferably, the substrate is water impermeable. Using a substrate prevents water loss from the personal care composition while the personal care composition is in contact with the keratinous tissue and thus prevents the personal care composition from drying out. Water loss from the personal care composition lowers the water concentration, thus increasing the concentration of active ingredients. This could result in irritation to the skin, which applicants wish to avoid.

The substrate preferably comprises at least one water impermeable material, and is preferably comprised of entirely water impermeable materials. The substrate material is preferably compatible with personal care compositions. Examples of useful water impermeable materials include but are not limited to polypropylene, polyethylene (including HDPE and LLDPE), polyethylene terephthalate, polyvinylchloride, polyamide, polycarbonate, polyurethane, cellulose acetate, polychloropene, polysulfone, polytetrafluoroethylene, polyvinyl acetate, polystyrene, polyphenylene oxide, acrylonitrile butadiene styrene, acrylic, acrylonitrile styrene acrylate, ethylene vinyl alcohol, natural rubber, latex, nylon, nitrile, silicone and thermo plastic elastomers. The substrate may comprise a single polymer or mixtures of polymers or copolymers. Preferably, the substrate comprises a plastic sheet, more preferably a plastic sheet comprising a polyolefin, even more preferably a polyethylene and even more preferably still high density polyethylene.

In an advantageous embodiment, the personal care composition is disposed upon the water impermeable material, preferably in the form of a plastic sheet, preferably comprising polyolefin, even more preferably polyethylene and even more preferably still high density polyethylene. In this advantageous embodiment, there is preferably no layer of water permeable material between the personal care composition and the water impermeable material. In a preferred embodiment, the water impermeable material forms a water impermeable layer.

The substrate may be provided in any suitable form such as a layer, web, film, foam, mixtures thereof or the like. The substrate may comprise one or more layers or films or foams or mixtures thereof, which may or may not be affixed to one another by means known in the art such as adhesion, lamination crimping for example.

The substrate advantageously possesses a rigidity in the range of from 5.00 g/cm to 0.08 g/cm, preferably from 3.00 g/cm to 0.08 g/cm, more preferably from 1.80 g/cm to 0.10 g/cm, even more preferably from 0.80 g/cm to 0.15 g/cm and even more preferably still from 0.60 g/cm to 0.25 g/cm. This rigidity of the substrate ensures that desirable handleability and conformability attributes of a personal care article are achieved. In particular, the article collapsing under gravity or folding is avoided, which is especially undesirable if different areas of the personal care composition are able to readily come into contact with each other, while maintaining the capability for the substrate to conform to the surface to which it is applied without folding or crinkling, in order to further improve depilatory efficiency. Accordingly, the substrate is readily conformable to the skin and/or hair without permanently deforming during use, as this may also result in problems for the user during application. In a preferred embodiment, the rigidity is substantially constant and does not change during the lifetime of a product.

Rigidity can be readily measured using the American Standard Test Method (ASTM) D2923-06, method B (i.e. using a powder to reduce the effect of static electricity) on a Handle-O-Meter, model #211-300, available from Thwing-Albert Instrument Co. of Philadelphia, Pa. The rigidity is read directly from the meter and expressed as grams per centimetre of sample width. Samples were prepared as 10.16 cm (4 inch) by 10.16 cm (4 inch) test specimens with edges parallel to the machine direction and transverse direction for substrates with directionality. Three rigidity measurements were determined on the same side of fresh test specimens orientated in the same substrate direction. A further three rigidity measurements were taken on the same side of fresh test specimens oriented at 90° to the first orientation. These six measurements were repeated on the opposite side to the first six measurements, on fresh test samples. The 12 rigidity measurements were then averaged and reported to 0.01 g/cm.

The rigidity of a substrate is a function of substrate thickness and inherent modulus of elasticity. Different materials have different moduli of elasticity. Based upon the material or materials that the substrate comprises, a substrate thickness should be selected that enables the desired rigidity of the substrate to be achieved. The substrate preferably has a mean substrate thickness of from 80 μm to 12 μm, more preferably from 50 μm to 15 μm, even more preferably

from 40 μm to 16 μm and even more preferably still from 30 μm to 17 μm.

Non-limiting examples of substrate material and thickness combinations for the substrate are:

| Substrate Material | Substrate Thickness [microns] | Rigidity [g/cm] |
| --- | --- | --- |
| HDPE | 13 | 0.13 |
| HDPE | 18 | 0.33 |
| HDPE | 36 | 1.05 |
| LLDPE | 23 | 0.23 |
| PP | 18 | 0.46 |
| [HDPE is a mixture of LBI 85% M6030 and Exxon Mobil 15% LD2001 manufactured on a Merritt-Davis casting line] [LLDPE is Exxon Mobil 15% LD2001 manufactured on a Merritt-Davis casting line] [PP is Basell PH835 manufactured on a Merritt-Davis casting line] | | |

The substrate may be a laminate comprising at least two materials, including non-wovens; paper; board; metal based substrates (eg aluminium foil); flocking or topical coatings (e.g. surfactants; printing); closed or open cell foams or substrates described herein above. In a preferred embodiment, at least one of the materials is water impermeable.

The substrate may comprise a textured or, alternatively, micro-structured surface on at least a portion of one side. Surface texturing or micro-structuring increases the effective surface area of the substrate and thus improves adherence of the personal care composition to said substrate, facilitating an easy removal of the personal care article by peeling it off the skin, or increases the grip of the surface, thus improving handleability. The textured structures may comprise dimples; lines or curvilinear embossments. A textured surface may be formed on the substrate by any appropriate technique, including embossment calendars and casting.

The substrate may be manufactured by any suitable method, including casting, injection moulding, co-injection moulding, over moulding, in-mold assembly, compression moulding, blow moulding, casting thermo or vacuum forming and where appropriate may be laminated by heat welding (which may further include the use of pressure, ultrasonic forces and radio or high frequencies), co-extrusion; adhesives, electro static adhesions (such as flocking by fibres) and topical surface applications.

Achieving a desired dose of personal care composition to the surface of the skin and/or hair is a further advantage of using a substrate-based product. However, if the substrate is able to stretch or tear, the personal care composition disposed upon it may be thinned, thickened or rupture in places, resulting in uneven and hence less desirable depilatory activity. In particular, low depilatory efficacy may result in areas treated with thinned or ruptured areas of the composition while higher depilatory efficacy and increased irritation may result in areas treated with thickened areas of the composition.

The potential problem of a substrate stretching may be avoided by selecting a substrate that does not permanently deform during use. This problem may also be avoided by selecting a substrate with a sufficiently high secant modulus such that it is less likely to stretch during normal use. Accordingly, in another preferred embodiment, the substrate has a secant modulus at 2% strain of greater than 689.5 bar (10,000 psi), more preferably greater than 1379.0 bar (20,000 psi), even more preferably greater than 2068.4 bar (30,000 psi) and even more preferably still greater than 2757.9 bar (40,000 psi) in order to achieve uniform application of the personal care composition to the surface of the body during usage. Without wishing to be bound by theory, applicants believe that using a substrate with an excessively low secant modulus at 2% strain can deform and thus break apart the personal care composition disposed on the substrate, leading to uneven depilatory action and increased risk of irritation. The secant modulus at 2% strain may be measured readily using the American Standard Test Method (ASTM)) 'Standard Test Method for Tensile Properties of Thin Plastic Sheeting D882-09' conducted on an MTS Insight1 Tensile Tester available from MTS Systems Co, Eden Prairie, MN, USA. This method may also be applied to non-plastic materials and is designed for use on sheets with a thickness of less than 1 mm.

The potential problem of a substrate tearing may be avoided by selecting a substrate that does not fail during usage. This problem may also be avoided by selecting a substrate with a sufficiently high nominal tensile strength such that it is less likely to tear during normal use. Accordingly, in another preferred embodiment, the substrate has a nominal tensile strength of at least 5 MPa more preferably at least 10 MPa even more preferably at least 15 MPa and even more preferably still at least 18 MPa in order to achieve uniform application of the personal care composition to the surface of the body during usage. Without wishing to be bound by theory, applicants believe that using a substrate with an excessively low nominal tensile strength can fail during usage and thus break apart the personal care composition disposed on the substrate, leading to uneven depilatory action and increased risk of irritation. The nominal tensile strength may be measured readily using the American Standard Test Method (ASTM) 'Standard Test Method for Tensile Properties of Thin Plastic Sheeting D882-09' conducted on an MTS Insight1 Tensile Tester available from MTS Systems Co, Eden Prairie, MN, USA. This method may also be applied to non-plastic materials and is designed for use on sheets with a thickness of less than 1 mm.

A layer of personal care composition can be applied to the surface of the substrate through any known technique of applying viscous fluids to substrates, including, for example, extrusion, casting (e.g., reverse roll, knife-over roll, slot die, Gravure roll), spraying, knife blade coating, and zone coating. Such techniques may be modified to alter the quantity of personal care composition disposed on the substrate. For example, the speed at which the substrate travels through an extrusion process determines the quantity of personal care composition disposed upon said substrate. The personal care composition may cover the entire first surface of the substrate or a portion thereof. Advantageously, the personal care composition covers less than the entire first surface of the substrate to facilitate handling. The substrate may comprise at least one region with two orthogonal dimensions each of a length greater than 1 cm, preferably greater than 1.5 cm and more preferably greater than 2 cm upon which no personal care composition is disposed.

In a preferred embodiment, the personal care composition is a depilatory composition and comprises a keratin reducing agent to weaken and/or break strands of unwanted hair. Non-limiting examples of suitable keratin reducing agents include: sulphide salts such as $Li_2S$, $Na_2S$, $K_2S$, MgS, CaS, SrS or BaS, hydrogen sulphide salts such as NaSH or KSH, thioglycol, thioglycerol, thioglycolamide, thioglycolhydrazide, thioglycolic acid, thioglycolate salts (such as potassium thioglycolate, calcium thioglycolate, ammonium thioglycolate, diammonium dithioglycolate, glyceryl monothioglycolate, or monoethanolamine thioglycolate), thiosalicylic acid, thiomalic acid, ammonium thiolactate, monoethanolamine thiolactate, dithioerythritol, 2-mercaptopropionic acid, 1,3-dithiopropanol, glutathione, dithiothreitol, cysteine, homocysteine, N-acetyl-L-cysteine and cysteamine. Advantageously, the keratin reducing agent is present in an amount of from 0.3% to 20%, preferably from 0.8% to 15%, more preferably from 1% to 10% by weight of the composition.

Advantageously, the depilatory composition may comprise at least one thioglycolate salt or thioglycollic acid acting as a hair removal agent when the depilatory composition is applied to unwanted hair. Preferably, the depilatory composition comprises sodium, potassium, magnesium, calcium, beryllium, strontium, zinc, monoethanolamine, ammonium, tetralkylammonium, imidazolium, pyridinium, phosphonium or glyceryl thioglycolate salts, or mixtures thereof, which may include dianion forms of thioglycolate. More preferably, the depilatory composition comprises at least one of sodium, potassium, magnesium or calcium thioglycolate, or mixtures thereof. Even more preferably the depilatory composition comprises potassium or calcium thioglycolate, or mixtures thereof. In a preferred embodiment, the concentration of the conjugate acid of the thioglycolate salt is from 0.5% to 12.0%, more preferably from 0.8% to 8.0% and even more preferably from 1.0% to 6.0% by weight of the depilatory composition.

In a preferred embodiment, the depilatory composition comprises a monovalent cation, preferably a monovalent metal cation. Without wishing to be bound by theory, the applicants believe that the presence of monovalent metal cations increases the dissociation of thioglycolate salts. The monovalent cations such as those derived from monovalent cation containing salts are able to displace the cation of the thioglycolate salt and further enhance dissociation of said thioglycolate salt. This increases the amount of deprotonated thioglycolate formed from the thioglycolate salt and therefore increases the effectiveness of the depilatory composition. Sources of monovalent cations include potassium, sodium, lithium, ammonium, tetraalkyl ammonium and imidazolium salts, which may be a component of another ingredient, for example a thickening system or skin care active. Preferred sources of monovalent cations include potassium and sodium salts.

In order to further enhance the safety of the resulting product, it is advantageous to limit the amount of monovalent cations, preferably monovalent metal cations, to which the skin is exposed when a depilatory composition is used, although a small quantity may improve the efficacy of the depilatory composition. Advantageously, the quantity of monovalent cations (or monovalent metal cations in the preferred embodiment above) per unit area of the aforementioned coated surface of the substrate is less than $5.10 \times 10^{-4}$ mol/cm$^2$, preferably less than $3 \times 10^{-4}$ mol/cm$^2$, more preferably from $1 \times 10^{-9}$ mol/cm$^2$ to $1.5 \times 10^{-4}$ mol/cm$^2$, even more preferably from $2.50 \times 10^{-8}$ mol/cm$^2$ to $6.65 \times 10^{-5}$ mol/cm$^2$ and even more preferably still from $6 \times 10^{-7}$ mol/cm$^2$ to $4.5 \times 10^{-5}$ mol/cm$^2$. The selection of keratin reducing agent and optional ingredients including the base may be made considering the quantity of monovalent cations or monovalent metal cations achieved.

Limiting the quantity of monovalent ion present in the depilatory composition may prevent skin irritation but also limits the quantity of thioglycolate salt that may be present in a formula, if monovalent ions containing thioglycolate salts or bases are used. Accordingly, in an advantageous embodiment, the depilatory composition comprises a divalent cation, preferably a divalent metal cation, and preferably wherein the thioglycolate salt, the buffering base (if present) or both comprises a divalent cation, or more preferably a divalent metal cation in order to enable the inclusion of additional depilatory active. In another preferred embodiment, the thioglycolate salt comprises a divalent metal cation. Applicants have established that thioglycolate salts comprising monovalent metal cations, such as potassium thioglycolate, are effective at removing hair from the skin, even at low doses, but may expose the skin tissue to harsh chemical conditions, resulting in irritation. On the other hand, thioglycolate salts comprising divalent metal cations, such as calcium thioglycolate, are relatively non-irritating to the skin.

In a depilatory composition comprising a mixture of monovalent and divalent ions, controlling the ratio of divalent ions to monovalent ions may also improve the safety characteristics of the personal care articles of the present invention. Increasing the concentration of divalent ions relative to the concentration of monovalent ions increases the likelihood that any particular depilatory active species is associated with a divalent ion, rather than the more irritating monovalent ions. On the other hand, increasing the concentration of monovalent ions increases the effectiveness of the depilatory composition. Accordingly, in an alternative embodiment the ratio of the concentration of divalent ions to the concentration of monovalent ions present in the depilatory composition is advantageously in the range of from 400:1 to 0.02:1, preferably from 200:1 to 0.1:1, more preferably 60:1 to 0.3:1, even more preferably from 20:1 to 0.5:1, and even more preferably still from 15:1 to 1:1.

The pH of the depilatory composition may advantageously be in the range of from 6 to 13.8, preferably from greater than 7 to 13, more preferably from 9 to 12.9, even more preferably from 10 to 12.8, even more preferably still from 12 to 12.7 and yet more preferably from 12.3 to 12.6 to improve the efficacy of the active ingredient. The depilatory composition may, in a preferred embodiment, comprise at least one base to control the pH. Preferably, the depilatory composition comprises potassium hydroxide; sodium hydroxide; lithium hydroxide; calcium hydroxide; barium hydroxide; caesium hydroxide; sodium hydroxide; ammonium hydroxide; strontium hydroxide; rubidium hydroxide; magnesium hydroxide; zinc hydroxide; sodium carbonate; pyridine; ammonia; alkanolamides (including monoethanolamine, diethanolamine, triethanolamine), phosphates (including tetrasodium phosphate), arginine or mixtures thereof.

More preferably, the depilatory composition comprises at least one buffering base, even more preferably the depilatory composition comprises calcium hydroxide, magnesium hydroxide; barium hydroxide; strontium hydroxide; zinc hydroxide; arginine or mixtures thereof. Still more preferably the depilatory composition comprises calcium hydroxide; magnesium hydroxide, zinc hydroxide, sodium hydroxide, potassium hydroxide or mixtures thereof. Even more preferably still, the depilatory composition comprises calcium hydroxide.

In a preferred embodiment, the depilatory composition comprises a buffering base that is present at a concentration of from 0.1% to 10.0%, more preferably from 0.5% to 8.0% and even more preferably from 1.0% to 5.0%, by weight of the depilatory composition.

In another preferred embodiment, the depilatory composition comprises at least one silicate or silica, advantageously at least one water-soluble or colloid-forming silicate or silica.

Preferably, the depilatory composition comprises at least one water-soluble or colloid-forming silicate selected from lithium silicates; sodium silicates (including disodium metasilicate pentahydrate and disodium metasilicate nanohydrate); potassium silicates; calcium silicates, ammonium silicates; manganese silicates; imidazolium silicates, synthetic and natural silicates (clays) or mixtures thereof. More preferably, the depilatory composition comprises at least one water-soluble or colloid-forming silicate selected from synthetic clays; sodium silicates, potassium silicates, or mixtures thereof and even more preferably the depilatory composition comprises a sodium silicate or mixtures of sodium silicates.

Alternatively, the depilatory composition comprises a form of silica that is colloid-forming, (such as amorphous microporous silica), forms sol or gel systems, (such as silica gels and nano-colloidal silicas), or is mesostructured. Surface modification of silica may be advantageous to promote the formation of stable colloid systems. Suitable synthetic and natural silicates (clays) are available commercially as: Laponite® RDS; XLS and S etc. (available from RockWood Additives Limited); Wyoming Bentonite; Californian Hectorite; Jadeite; Enstaite and Rhodonite; Benonate® EW (available from Rheox Inc.); Bentolite® (available from Southern Clay Products Inc.) Optigel® (available from Süd Chemie Rheologicals).

The silicate or silica is preferably present in the depilatory composition in an amount per unit area of the coated region of from $2.05 \times 10^{-8}$ mol/cm$^2$ to $1.23 \times 10^{-4}$ mol/cm$^2$, preferably from $1.64 \times 10^{-7}$ mol/cm$^2$ to $3.69 \times 10^{-5}$ mol/cm$^2$ and more preferably from $4.92 \times 10^{-7}$ mol/cm$^2$ to $8.20 \times 10^{-6}$ mol/cm$^2$. Within the preferred ranges, the effectiveness of the depilatory composition is further increased while irritation is maintained within an acceptable level. Without wishing to be bound by theory, applicants believe that an amount of silicate or silica is required in order to enhance the dissociation of the thioglycolate salt sufficiently for the increase in efficacy to be clearly apparent to the user, but that excessive dose of silicate or silica may lead to over-dissociation of the thioglycolate salt resulting in increased skin irritation. Alternatively, the silicate or silica may be present in the depilatory composition in an amount of from 0.01% to 5%, preferably 0.1% to 4%, more preferably 0.2% to 3% and even more preferably from 0.5% to 2% by weight of the depilatory composition.

An accelerant may be employed in the depilatory composition. This optional component accelerates the rate of depilatory action of the depilatory agent. Suitable accelerants include, but are not limited to, urea; thiourea; dimethyl isosorbide; arginine salts; ethoxydiglycol; propylene glycol and methylpropyldiol. The accelerant may be present in a concentration range of from 0.5% to 10%, more preferably from 2% to 8% and even more preferably from 2% to 5% by weight of the depilatory composition.

The personal care composition may optionally comprise a thickening agent. A representative but not exhaustive list can be found in "The Encyclopaedia of Polymers and Thickeners for Cosmetics" compiled and edited by Robert Y. Lochhead, PhD and William R. Fron, Department of Polymer Science, University of Southern Mississippi. Exemplary classes of thickening agents include gums, carbomers, polymers and copolymers of acrylic acid, associated thickeners, layered silicates/clays and natural polymers (including polysaccharides). One or more thickening agents may be included in the depilatory composition. It may be desirable to utilize gel network structures or oil-in-water emulsions to thicken the depilatory compositions. Suitable materials for preparing the gel network structures or oil-in-water emulsions are well represented in the art and include fatty materials such as fatty alcohols (for example cetyl alcohol and stearyl alcohol) alone or used in conjunction with non-polar oils such as paraffin or mineral oils. An appropriate emulsifier may also be used to form and stabilize the bilayer structure characteristic of gel network structures or to form and stabilize an oil-in-water emulsion. The thickening agent may be present at a level of from about 0.01% to about 20%, preferably from about 0.1% to about 10%, more preferably from about 0.3% to about 5%, and even more preferably from about 0.5% to about 4%, by weight of the personal care composition.

Advantageously, the thickening agent comprises carrageenan. The carrageenan is preferably present in an amount of from 0.1% to 10%, more preferably from 0.5% to 8%, even more preferably from 1% to 5% and even more preferably still from 2% to 4% by weight of the depilatory composition. The carrageenan may be iota, kappa or lambda carrageenan, and in a preferred embodiment is iota carrageenan. Without wishing to be bound by theory, the applicants believe that a personal composition comprising carrageenan has both an affinity to the surface of the skin and/or hair, providing an effect analogous to a frictional resistance opposing spreading of the composition and cohesive forces that further prevent spreading and additionally prevent rupturing of the composition.

The personal care composition may include skin care and or hair care ingredients such as conditioning agents selected from the group consisting of humectants, moisturizers, or skin conditioners (including mineral oil; almond oil; chamomile oil; jojoba oil; avocado oil; shea butter, niacinamide and glycerine); skin rejuvenation compositions (for example targeted for fine lines, wrinkles and uneven skin tone including retinoids), cosmetic compositions; antiinflammatory agents (including corticosteroids); anti-oxidants (including flavonoids) radical scavengers; sunscreen agents; skin cooling or warming agents and the like. The personal care composition may comprise one or more skin care and or hair care ingredients present in an amount of from about 0.001% to about 10%, more preferably from about 0.01% to about 7%, and even more preferably from about 0.025% to about 5%, by weight of the depilatory composition. The personal care composition may further comprise components known, conventionally used, or otherwise effective for use in personal care and or haircare compositions particularly dyes; pigments (including ultra marines and talc); anionic, cationic, non-ionic and/or amphoteric or zwitterionic surfactants, polymers (including hydrophobically modified polymers); dispersing agents; solvents; lubricants; fragrances; preservatives; chelants, proteins and derivatives thereof, plant materials (e.g. aloe, chamomile and henna extracts); silicones (volatile or non-volatile, modified or non-modified); film-forming agents; film forming promoters and mixtures thereof.

Personal care articles of the present invention may take any form suitable for applying to skin and/or hair. The size and shape of the personal care article may take any form suitable for application to the body area from which hair is to be removed. The personal care article will preferably relate to the body area or zone from which hair is to be removed, especially the face (including the jaw, chin and upper lip regions of the face), underarm and bikini areas. Preferably, the personal care article takes the form of a mask (configured for the face) or a strip/patch (configured for general use). In another preferred embodiment, the substrate of the personal care article is substantially planar.

For applications intended for use on the human upper lip skin, the at least one portion of personal care composition of the first surface of the substrate, preferably comprises an upper-lip portion adapted to be placed above a human mouth, and a first return portion projecting from the upper lip portion and adapted to be placed contiguously with the outer extremity of the vermilion lip in a first corner of the mouth. The first return portion has a length along its greatest dimension of at least 0.2 cm, preferably from 0.5 cm to 5 cm, more preferably from 0.75 cm to 4 cm, even more preferably from 1 cm to 3 cm. Applicants have found that this configuration enables the user to remove unwanted hair from the skin immediately surrounding the corner of the mouth while lowering the risk of personal care composition contacting the vermillion lip, where it may cause irritation. In an alternative embodiment, the portion of personal care composition of the first surface of the substrate further comprises a second return portion projecting from the upper lip portion and adapted to be placed contiguously with the outer extremity of the vermillion lip in a second corner of the mouth.

Advantageously, the upper lip portion has a length along its greatest dimension of at least 0.2 cm, preferably from 0.5 cm to 15 cm, more preferably from 1 cm to 12 cm, even more preferably from 2 cm to 10 cm and even more preferably still from 3 cm to 8 cm. This dimension enables the upper lip portion to cover a desirable length of the upper lip and thus achieve the desired depilatory action. In a preferred embodiment, the upper lip portion is adapted to be placed to be at least partially contiguously with the upper border of the upper vermilion lip, to enable depilatory action to be achieved on the skin immediately surrounding the upper vermilion lip while lowering the risk of personal care composition contacting the upper vermilion lip, where it may cause irritation.

In another preferred embodiment, the portion of personal care composition comprises a lower lip portion adapted to be placed below a human mouth, preferably wherein the lower lip portion is adapted to be placed to be least partially contiguously with the lower border of the lower vermilion lip to enable depilatory action to be achieved on the skin immediately surrounding the lower vermilion lip while lowering the risk of personal care composition contacting the lower vermilion lip, where it may cause irritation.

Personal care articles of the present invention may comprise at least two finger-tabs being substantially free of personal care composition and positioned on substantially opposing sides of the portion of personal care composition of the first surface of the substrate. These finger tabs enable a user to apply tension to the coated portion of the substrate. Surprisingly, applicants have found that applying tension across the coated portion of the personal care article creates an effect of temporarily causing the coated region to exhibit an apparent increased rigidity, enabling the user to accurately position the coated region, and hence personal care composition on to the desired region of the body. Tensioning the coated portion may be achieved in a number of ways, non-limiting examples of which include holding the personal care article either side of the coated portion , for example with the hands or a tool, so as to apply tension between the areas being held. Alternatively, personal care articles of the present invention may comprise at least one finger-tab being substantially free of personal care composition and positioned to allow the weight of the article to tension the coated portion when being held by the finger-tab.

In a preferred embodiment, at least one finger tab extends from the perimeter of the coated portion by a minimum of 1 cm, preferably from 1.5 cm to 5 cm, more preferably from 2 cm to 4 cm and even more preferably from 2.5 cm to 3.5 cm. In another preferred embodiment, both finger-tabs extend from the perimeter of the coated portion by a minimum of 1 cm, preferably from 1.5 cm to 5 cm, more preferably from 2 cm to 4 cm and even more preferably from 2.5 cm to 3.5 cm, in order to aid handling of the personal care article.

Personal care articles of the present invention may comprise a protective release layer removably attached to the personal care composition, preferably on a surface of the personal care composition substantially opposing that which is in contact with the substrate. The protective release layer may comprise materials including polymer resins such as a polyolefins e.g. polypropylene (including stratified biaxially oriented polypropylene), polyethylene (including LDPE; LLDPE; HDPE; Metallocene) or polyethylene terephthalate. Alternative materials which may be used include polyvinylchloride, polyamide, acetyl, acrylonitrile butadiene styrene, acrylic, acrylonitrile styrene acrylate, ethylene vinyl alcohol, ethylene vinyl acetate, Nylon, Latex, natural or synthetic rubbers, polycarbonate, polystyrene, silicone or thermo plastic elastomer, thermo plastic vulcanate or copolymers of said materials. Where appropriate the protective release layer may comprise one or more laminations, combinations of multiple layers and/or indications (which may include instructions and illustrations) relating to at least one aspect of the usage of the personal care article. In an advantageous the protective release layer may comprise a coating of a non-stick material. Exemplary non-stick coatings include wax, silicone, fluoropolymers such as TEFLON®, and fluorosilicones. In a preferred embodiment, the protective release layer covers at least the entire aforementioned coated region of the substrate. In another preferred embodiment the protective release layer is water impermeable. In a further preferred embodiment, the protective release layer has a mean substrate thickness of at least 85 microns, more preferably from 85 microns to 130 microns, even more preferably from 90 microns to 120 microns. In yet another preferred embodiment, the protective release layer extends beyond the coated region of the substrate to provide a removal tab.

In a preferred embodiment, the personal care articles of the present invention are packaged to prevent water loss and/or oxygen permeation. Alternatively, the personal care articles of the present invention are packaged in water impermeable packaging. Examples of suitable packaging materials include films of ethylene vinyl alcohol; polyethylene; polypropylene; Nylon; foil laminates (including metalized polyethylene terephthalate; biaxially oriented polypropylene (SBOPP)), mixtures thereof, laminates thereof or multi-laminates thereof. More preferably, the packaging comprises an inert gas and even more preferably the inert gas comprises at least one of nitrogen, argon or carbon dioxide. Alternatively, the packaging comprises a partial vacuum.

A second aspect of the invention relates to a method of treating the hair or skin, preferably removing hair from the skin, comprising the steps of:

(a) applying a personal care article according to the present invention to the surface of the skin, preferably mammalian and more preferably human skin,
(b) leaving said personal care article in contact with the skin for a period of at least 1 minute, preferably from 2 to 10 minutes, more preferably from 2 to 8 minutes,
(c) removing said personal care article from the surface of the skin, and
(d) preferably rubbing, scraping, rinsing or wiping the surface of the skin in the area to which the personal care article was applied.

The period of contact of the personal care article with the skin may be varied depending on the on the nature of the personal care composition coated on the substrate of the personal care article and may be applied for time periods of up to 45minutes, preferably up to 30minutes.

Advantageously, the method of removing hair from the skin further comprises the step of tensioning the coated portion of the personal care article prior to applying it to the skin.

The same means used to apply tension to the coated portion may be used to ensure that the personal care article is applied to the surface of the body such that the coated portion is applied under tension to the unwanted hair in order to maintain the improved handling characteristics described above. In a preferred embodiment, the tension is kept substantially constant during application of the personal care article. The flexible nature of the substrate allows the substrate to conform to the surface of the body to offer improved contact between the personal care composition and the unwanted hair. In a preferred embodiment, the tension may be at least partially, more preferably substantially completely released from the coated portion after applying the personal care article to the skin in order to improve the conformability of the personal care article.

A third aspect of the invention is related to a depilatory kit, preferably a depilatory, which comprises the personal care article of the present invention, packaging for said personal care article, and at least one of a third component selected from:

a) a pre-treatment skin care composition which may comprise ingredients to promote skin conditioning (e.g. emollients), hair hydration or provide a skin barrier (e.g. hydrophobic materials) and intended for use prior to applying the personal care article.

b) a post-treatment skin care composition which may comprise ingredients to promote skin conditioning; moisturizers, skin rejuvenation compositions (targeted for fine lines, wrinkles and uneven skin tone, for example), cosmetic compositions (e.g., foundation, rouge), sunscreens and the like as described herein above. The complementary post treatment skin care compositions may be leave-on or rinse-off compositions. The skin care compositions may also be designed to immediately follow application of the hair removal products. For example, a finishing composition may be applied to the same skin area to combat lingering odour and irritation caused by residual depilatory agent. The finishing composition may comprise a metal oxide (e.g., zinc oxide, aluminum oxide, and magnesium oxide) that is capable of complexing with any remaining depilatory agent remaining on the targeted skin area to reduce continued odour and subsequent skin irritation.

c) a tool to assist in the removal of hair and/or personal care composition from the skin.

d) indications (which may include instructions and/or illustrations) relating to at least one aspect of usage of the personal care article or another component of the kit.

The following example further describes and demonstrates one embodiment within the scope of the present invention. The example is given solely for the purpose of illustration and is not to be construed as a limitation of the present invention, as many variations thereof are possible.

**Measuring the median particle diameter D(v,0.5)**

The particle size distribution was measured on a Malvern Instruments Mastersizer 2000 with the Scirocco dry dispersion accessory. The measurements were carried out in accordance with the principles of ISO13320 "Particle Size Analysis - Laser Diffraction Methods". The median particle diameter D(v,0.5) was reported as an average of a minimum of 10 individual internal replicates to the nearest 0.1 micron.

**Preparation of personal care composition examples**

The compositions in table 1 were separately prepared in a sanitized 400 ml speed mixer plastic pot by adding DI water followed by the Calcium Hydroxide and Sodium Silicate then the slow addition of Carrageenan over a period of 10 minutes with mixing (increasing mixing speed if required). After a further 10 minutes of mixing, the Calcium thioglycolate was then added and the batch continued to be mixed for a further 10 minutes. The batch was then transferred to a thick walled 400 ml glass beaker and milled for 2 minutes using an IKA T50 (5,200 rpm). In compositions comprising the particles, the particles were then added and the batch was mixed for a further 10 minutes before storage. The amounts of Carrageenan and DI water were slightly adjusted as needed to maintain the same rheological properties of each composition. This ensures the single variable comparisons can be made between the inventive and comparative examples based on the particles.

**Table 1: Personal care composition examples**

| Composition examples: [%wt] | 1 | 2 | 3 | 4 | 5 | 6 | 7 | 8 |
|---|---|---|---|---|---|---|---|---|
| DI water | 83.57 | 83.57 | 83.57 | 83.57 | 87.05 | 83.57 | 86.61 | 83.57 |
| Calcium hydroxide [a] | 2.88 | 2.88 | 2.88 | 2.88 | 3.00 | 2.88 | 2.99 | 2.88 |

(continued)

| Composition examples: [%wt] | 1 | 2 | 3 | 4 | 5 | 6 | 7 | 8 |
|---|---|---|---|---|---|---|---|---|
| Sodium silicate solution (42% w/w in water) [b] | 1.68 | 1.68 | 1.68 | 1.68 | 1.75 | 1.68 | 1.74 | 1.68 |
| Carrageenan Cl-123[c] | 2.40 | 2.40 | 2.40 | 2.40 | 2.50 | 2.40 | 2.49 | 2.40 |
| Calcium thioglycolate trihydrate [d] | 5.47 | 5.47 | 5.47 | 5.47 | 5.70 | 5.47 | 5.67 | 5.47 |
| Particles amount | 4.00 | 4.00 | 4.00 | 4.00 | 0.00 | 4.00 | 0.50 | 4.00 |
| Particle material [e] | Inhance UH-1080 | Inhance UH-1080 (Sieved 160$\mu$m to 250$\mu$m) | Inhance UH-1080 (Sieved 38$\mu$m to75$\mu$m) | Micropoly 1160S | N/A | Asensa SC220 (Sieved 355$\mu$m-to 500$\mu$m) | Inhance UH-1080 | Micropoly 250S |
| Median particle diameter[f] D(v, 0.5) [$\mu$m] | 117.5 | 155.5 | 60.6 | 15.0 | N/A | 415.3 | 117.5 | 2.5 |

[a] Calcium hydroxide Reag. Ph. Eur. puriss. p.a. available from Sigma-Aldrich Co.
[b] Sodium silicate solution (42% w/w in water) available from Cognis.
[c] Carrageenan Cl-123 available from CPKelco.
[d] Calcium thioglycolate trihydrate 99.8% available from BRUNO BOCK Chemische Fabrik GmbH & Co.
[e] Polyethylene particles. For Compositions 1 and 7, Inhance UH-1080 available from Inhance Fluoro Seal Ltd was used as supplied. For Composition 2, Inhance UH-1080 available from Inhance Fluoro Seal Ltd was sieved to obtain the particle diameter fraction between 160 microns to 250 microns. For Composition 3, Inhance UH-1080 available from Inhance Fluoro Seal Ltd was sieved to obtain the particle diameter fraction between 38 microns to 75 microns. For Composition 4, Micropoly 1160S available from Micro Powders, Inc. was used as supplied. For Composition 8, Micropoly 250S available from Micro Powders, Inc. was used as supplied. For Composition 6, Asensa SC 220 available from Honeywell International was sieved to obtain the particle diameter fraction between 355 microns to 500 microns.
[f] Median particle diameter D(v,0.5) was measured as described herein above on a Malvern Instruments Mastersizer 2000 with the Scirocco dry dispersion accessory.

**Inventive and comparative personal care article examples and robustness test method**

A rectangular test area of 1.5cm in width and 3.5cm in length was clearly marked onto KMS1 substrate (a cast polypropylene film, 17$\mu$m in thickness, available from Toray Industries Inc. that had been cut to 30cm in length and 10cm in width) such that the test area length is centered and perpendicular to the width of the KMS1 and 2cm from one end of the KMS1 along the length of the KMS1. The compositions 1 to 7 above were disposed to a mean composition thickness of 380 microns on the high surface energy side of the KMS1 substrate, as a rectangle of width 1.5cm and length 3.5cm covering the test area using a stencil and wiper blade. The stencil was left in place for 30 seconds after the composition had been disposed before the stencil was removed.

The disposed composition was left for a further 30 seconds. The flexible SBOPP 9741 release liner film (cast polypropylene film, 89$\mu$m in thickness, available from 3M cut to 30cm in length and 10cm in width) was carefully placed onto the composition such that the treated surface of the SBOPP 9741 was in contact with the composition and both the SBOPP 9741 and KMS1 were completely overlaid along their lengths and widths. Care was taken not to disturb the composition during the procedure. After a further 30 seconds, the combined KMS1, composition and SBOPP 9741 were carefully placed onto a smooth surface that was fixed at an incline of 1.15°, such that the length of the substrates was parallel to the incline direction where the KMS1 substrate is in contact with the smooth surface. A cylinder measuring 21.5cm in length with a diameter of 6.35cm, and weighing 5.75kg, was then positioned such that its length was centered and parallel to the width of the substrates and placed on the smooth surface held at rest a distance of 13.5cm away from the highest edge of the composition up the inclined surface. The cylinder was then released from rest and allowed to roll over the release liner and along the length of the composition. The area of displaced composition was measured to the nearest $0.01cm^2$ and the experiment was then repeated three times on new freshly prepared test areas and substrates with the same composition and thickness of the disposed composition and the average recorded to the nearest $0.01cm^2$ The percentage dose reduction of the composition was then calculated according to equation 1 and reported to the nearest 0.1%.

The above procedure was repeated with compositions 1, 3, 4, 5 and 8 disposed to a mean composition thickness of 89 microns on the high surface energy side of the KMS1.

**Equation 1: Personal care article robustness test method dose reduction Equation**

$$\text{Dose Reduction }[\%] = 100 - \left( \frac{\text{Start composition test area}}{\text{Average area of displaced composition}} \times 100 \right)$$

wherein the start composition test area is $5.25cm^2$

A personal care article that has a dose reduction of greater than 90% as a result of damage is considered not to achieve the desired effectiveness and also may result in undesired effects such as irritation, messiness and difficulties in handling and conformability of the personal care article to the skin.

**Inventive and comparative personal care article robustness test results**

Table 2 below shows the results of the personal care article robustness test method for the inventive example A and comparative example E, where the mean composition thickness is 380 microns and inventive example H and comparative example J, where the mean composition thickness is 89 microns

**Table 2: Results for the robustness test**

| Example | Inventive example A | Comparative example E | Inventive example H | Comparative example J |
|---|---|---|---|---|
| Example composition | 1 | 5 | 3 | 5 |
| Mean composition thickness [μm] | 380 | 380 | 89 | 89 |
| Displacement area [a] [cm²] | 23.11 | 163.99 | 12.78 | 89.69 |
| Dose reduction [b] [%] | 77.3 | 96.8 | 58.9 | 94.1 |
| [a] Displacement area of the personal care composition was measured according to the personal care article robustness test method described herein above. | | | | |
| [b] Dose reduction was calculated according to Equation 1 described herein above. | | | | |

The results in Table 2 clearly demonstrate the advantages of inventive example A, comprising a personal care composition with particles, which has an acceptable dose reduction of less than 90% after the robustness test. This is due to a relatively small increase in the displaced area of composition.

In comparative example E, where the composition does not contain particles, the displaced area of composition is $163.99cm^2$ and therefore the personal care composition has an unacceptable dose reduction of 96.8%. Comparative example E is therefore considered not to achieve the desired effectiveness and may give rise to undesired effects such as messiness and irritation.

The results in Table 2 also demonstrate that the advantages of the personal care composition comprising particles are also achieved with different mean composition thicknesses (89 microns).

Table 3 below shows the results of the personal care article robustness test method for the inventive (A, B and C) and comparative (D, F and G) examples where the mean composition thickness is 380 microns.

The inventive (A, B and C) examples are preferred embodiments and comprise compositions where the particles percentage of mean composition thickness meets the preferred as described herein above.

Comparative example D comprises a composition where the particles percentage of mean composition thickness is below the preferred percentage (<10%).

Comparative example F comprises a composition where the particles percentage of mean composition thickness is above the preferred percentage (>100%).

Comparative example G comprises a composition with the preferred particles percentage of mean composition thickness, but below the preferred particle concentration (<1%).

**Table 3: Results for the robustness test (mean composition thickness 380 microns)**

| Example | Inventive examples | | | Comparative examples | | |
|---|---|---|---|---|---|---|
| | **A** | **B** | **C** | **D** | **F** | **G** |
| **Example composition** | 1 | 2 | 3 | 4 | 6 | 7 |
| **Particles percentage of mean composition thickness [a] [%]** | 31 | 41 | 16 | 4 | 109 | 31 |
| **Particles concentration [% w/w]** | 4.0 | 4.0 | 4.0 | 4.0 | 4.0 | 0.5 |
| **Displacement area [b] [cm$^2$]** | 23.11 | 18.44 | 41.79 | 126.10 | 36.53 | 136.95 |
| **Dose reduction [c] [%]** | 77.3 | 71.5 | 87.4 | 95.8 | 85.6 | 96.2 |

[a] particles percentage of the mean composition thickness can be calculated by dividing the median particle diameter D(v,0.5), as measured on a Malvern Instruments Mastersizer 2000 with the Scirocco dry dispersion accessory, by the mean composition thickness and multiplying by 100.
[b] Displacement area of the personal care composition was measured according to the personal care article robustness test method described herein above.
[c] Dose reduction was calculated according to Equation 1 described herein above.

The results in Table 3 clearly demonstrate that the preferred embodiments of the personal care articles meet the preferred requirements described herein above for a mean composition thickness of 380 microns, as is the case of inventive examples (A, B and C), the dose reductions of the personal care compositions after the robustness test are acceptable as being less than 90%. This is due to relatively small increase in the displaced area of composition.

In comparative example D, while the composition does contain particles, the particle percentage of mean composition thickness is less than 10% and as a result the personal care articles is not robust towards damage with an unacceptable dose reduction of 95.8% after the test.

In the case of comparative example F, while the dose reduction is less than 90%, both the starting and displaced mean composition thickness are uneven due to the size of the particles being greater than the mean composition thickness. This is considered to result in undesired effectiveness due to the uncontrolled dose. Furthermore, in cases like comparative example F, where the particle percentage of mean composition thickness is greater than 100%, the personal care article is not only challenging to manufacture, but also looks and feels unacceptable as a personal care article.

In comparative example G, the preferred particle percentage of mean composition thickness is met (the particle being the same material as used in inventive example A), but the particle concentration is less than the preferred level (<1%). This provides insufficient robustness of the personal care article and results in an unacceptable dose reduction of 96.2%.

Table 4 below, shows the results of the personal care article robustness test method for the inventive (H and I) and comparative (K and L) examples, where the mean composition thickness is 89 microns. The inventive (H and I) examples are preferred embodiments and comprise compositions where the particles percentage of mean composition thickness meets the preferred requirements as described herein above.

Comparative example K comprises a composition where the particles percentage of mean composition thickness is below the preferred percentage (<10%).

Comparative example L comprises a composition where the particles percentage of mean composition thickness is above the preferred percentage (>100%).

Comparative example F comprises a composition with the preferred particles percentage of mean composition thickness, but below the preferred particle concentration level (<1%).

**Table 4: Results for the robustness test (mean composition thickness 89 microns)**

| Example | Inventive examples | | Comparative examples | |
|---|---|---|---|---|
| | **H** | I | **K** | **L** |
| **Example composition** | 3 | 4 | 8 | 1 |
| **Particles percentage of mean composition thickness [a] [%]** | 68 | 17 | 3 | 132 |
| **Particles concentration [% w/w]** | 4 | 4 | 4 | 4 |
| **Displacement area [b] [cm$^2$]** | 12.78 | 28.97 | 69.12 | 9.08 |
| **Dose reduction [c] [%]** | 58.9 | 81.9 | 92.4 | 42.2 |

[a] particles percentage of the mean composition thickness can be calculated by dividing the median particle diameter D(v,0.5), as measured on a Malvern Instruments Mastersizer 2000 with the Scirocco dry dispersion accessory, by the mean composition thickness and multiplying by 100.

[b] Displacement area of the personal care composition was measured according to the personal care article robustness test method described herein above.

[c] Dose reduction was calculated according to Equation 1 described herein above.

The results in Table 4 clearly demonstrated that when the personal care article meets the preferred requirements described herein above for a mean composition thickness of 89 microns, as is the case of inventive (H and I) examples, the dose reductions of the personal care compositions after the robustness test are acceptable as being less than 90%. This is due to relatively small increase in the displaced area of composition. Furthermore, these examples demonstrate that the preferred requirements described herein above are true for different mean composition thicknesses (compare with table 3). Interestingly, the inventive example I (composition 4 disposed at 90 microns) has an acceptable dose reduction of 81.9% whereas comparative example D (composition 4 disposed at 380 microns) had an unacceptable dose reduction of 95.8%. This is due to the dependence on the mean composition thickness.

The comparative example L (composition 1 disposed at 90 microns) is unacceptable due to the uneven dose, feel and look and manufacturing challenges resulting from the particles percentage of mean composition thickness being greater than 100%. Whereas for comparative example A (composition 1 disposed at 380 microns) it is acceptable due to the dependence on the mean composition thickness. These examples supports the preferred embodiments that the particle percentage of mean composition thickness requirement as the personal care article robustness is dependent on the ratio of the median particle diameter D(v,0.5) and the mean composition thickness. The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

**Claims**

1. A personal care article comprising a substrate, said substrate having a first surface, said surface having at least a portion comprising a personal care composition disposed thereon, with a mean composition thickness of from 0.01 mm to 5.00 mm, wherein the personal care composition comprises from 1% to 30% by weight particles having less than 0.01% by weight of volatile material or fragrance having a boiling point at standard pressure below 200°C and wherein the particle percentage of mean composition thickness is from 10% to 100% calculated by dividing the mean particle diameter D(v,0.5) as measured on a Malvern Instruments Mastersizer 2000 with the scirocco dry dispersion accessory, by the mean composition thickness and multiplying by 100,
wherein said composition is aqueous, comprising water in an amount of greater than 40%.

2. A personal care article according to Claim 1, wherein said at least one portion of said personal care composition is disposed on the first surface of the substrate with a mean composition thickness of from 0.05mm to 2.50mm, even more preferably from 0.10mm to 1.00mm.

3. A personal care article according to any preceding Claim, wherein said particles have a concentration from 1.5% to 15.0% by weight and more preferably from 2.5% to 10.0% by weight of the personal care composition.

4. A personal care article according to any preceding Claim, wherein said particles are inert in the personal care composition and are preferably solid and more preferably selected from pulverized walnut husks, pulverized dried apricot seed, pulverized dried peach seed, powdered ceramics, glass, polymers natural rubber, latex, silicones, waxes, talc, tricalcium phosphate, clays, zeolite, or blends thereof, more preferably selected from polyethylene, polypropylene and mixtures thereof.

5. A personal care article according to any preceding Claim, preferably from 15% to 80%, even more preferably from 20% to 50%

6. A personal care article according to any preceding Claim, wherein said personal care composition has a yield point from 50 Pa to 2000 Pa, preferably from 65 Pa to 1200 Pa, more preferably from 80 Pa to 750 Pa when measured via a stress controlled amplitude sweep; at a frequency of 1Hz and a temperature of 25°C, wherein the yield is defined as the 5% decrease in magnitude of the elastic modulus G' plateau value as measured on a TA1000 Rheometer, available from TA Instruments of New Castle, Delaware, USA.

7. A personal care article according to any preceding Claim, wherein said particles have a hardness according to the Moh's scale of less than 5, more preferably less than 3 and even more preferably less than 2,

8. A personal care article according to any preceding Claim, wherein said personal care composition comprising water in an amount of from 50% to 98%, more preferably from 60% to 95% by weight of the personal care composition.

9. A personal care article according to any preceding Claim, wherein said substrate has a rigidity in the range of from 5.00 g/cm to 0.08 g/cm, preferably from 3.00 g/cm to 0.08 g/cm, more preferably from 1.80 g/cm to 0.10 g/cm, even more preferably from 0.80 g/cm to 0.15 g/cm, as determined by ASTM D2923-06 Method B.

10. A personal care article according to any preceding Claim, wherein said particles are shaped regular, irregular and mixtures thereof, preferably selected from spherical, elliptical, tetrahedron, octahedron, cube, dodecahedron (pentagonal faces), icosahedron (triangular faces) shaped particles and mixtures thereof, more preferably irregular shape.

11. A personal care article according to any preceding Claim, wherein said substrate comprises a material and said material is water impermeable, preferably the substrate comprises a plastic sheet, more preferably the plastic sheet comprises a polyolefin, more preferably a polyethylene and even more preferably high density polyethylene.

12. A personal care article according to any preceding Claim, further comprising a protective release layer removably attached to the personal care composition, preferably on a surface of the personal care composition not in contact with the substrate.

13. A personal care article according to any preceding Claim, wherein the personal care composition comprises a reducing agent, preferably thioglycolic acid or a thioglycolate salt, preferably present in an amount of from 0.3% to 20%, preferably from 0.8% to 15%, more preferably from 1% to 10% by weight of the personal care composition.

14. A method of treating the skin or hair, preferably removing hair from the skin, comprising the steps of:

   (a) applying a personal care article according to any preceding Claim to the surface of the skin, preferably mammalian and more preferably human skin,
   (b) leaving said personal care article in contact with the skin for a period of greater than 1 minute, preferably from 2 to 10 minutes, more preferably from 2 to 8 minutes,
   (c) removing said personal care article from the surface of the skin, and

(d) preferably rubbing, scraping, rinsing or wiping the surface of the skin in the area to which the personal care article was applied.

15. A personal care kit, preferably a depilatory kit, comprising:

(a) A personal care article according to any one of Claims 1-13,
(b) At least one of a pre-treatment skin care composition, a post-treatment skin care composition and/or a tool to assist removal of hair and/or personal care composition after use, and
(c) Packaging for said kit.

**Patentansprüche**

1. Körperpflegeartikel, ein Substrat umfassend, wobei das Substrat eine erste Oberfläche aufweist, wobei die Oberfläche mindestens einen Abschnitt aufweist, der eine darauf angeordnete Körperpflegezusammensetzung mit einer mittleren Dicke der Zusammensetzung von 0,01 bis 5,00 mm aufweist, wobei die Körperpflegezusammensetzung von 1 Gew.% bis 30 Gew.-% Partikel umfasst, die weniger als 0,01 Gew.-% eines flüchtigen Materials oder Duftstoffes mit einem Siedepunkt bei Normaldruck von unter 200 °C aufweisen, und wobei der Prozentsatz an Partikeln von der mittleren Dicke der Zusammensetzung von 10 % bis 100 % beträgt, berechnet aus der Division des mittleren Partikeldurchmessers $D(v,0,5)$,
gemessen mit einem Malvern Instruments Mastersizer 2000
mit der Scirocco Trockendispergiereinheit, durch die mittlere Dicke der Zusammensetzung und Multiplikation mit 100, wobei die Zusammensetzung wässrig ist, wobei sie Wasser in einer Menge von mehr als 40 % umfasst.

2. Körperpflegeartikel nach Anspruch 1, wobei der mindestens eine Abschnitt der Körperpflegezusammensetzung auf der ersten Oberfläche des Substrats mit einer mittleren Dicke der Zusammensetzung von 0,05 mm bis 2,50 mm, noch bevorzugter von 0,10 mm bis 1,00 mm, angeordnet ist.

3. Körperpflegeartikel nach einem der vorstehenden Ansprüche, wobei die Partikel eine Konzentration von 1,5 Gew.-% bis 15.0 Gew.-%, und mehr bevorzugt von 2,5 Gew.-% bis 10,0 Gew.-%, der Körperpflegezusammensetzung aufweisen.

4. Körperpflegeartikel nach einem der vorstehenden Ansprüche, wobei die Partikel in der Körperpflegezusammensetzung reaktionsträge sind und vorzugsweise fest sind und mehr bevorzugt ausgewählt sind aus pulverisierten Walnussschalen, pulverisierten getrockneten Aprikosenkernen, pulverisierten getrockneten Pfirsichkernen, Keramikpulver, Glas, Polymeren Naturkautschuk, Latex, Silikonen, Wachsen, Talkum, Tricalciumphosphat, Tonerden, Zeolith, oder Mischungen davon, mehr bevorzugt ausgewählt aus Polyethylen, Polypropylen und Mischungen davon.

5. Körperpflegeartikel nach einem der vorstehenden Ansprüche, vorzugsweise von 15 % bis 80 %, noch mehr bevorzugt von 20 % bis 50 %

6. Körperpflegeartikel nach einem der vorstehenden Ansprüche, wobei die Körperpflegezusammensetzung eine Fließgrenze von 50 Pa bis 2000 Pa, vorzugsweise von 65 Pa bis 1200 Pa, mehr bevorzugt von 80 Pa bis 750 Pa aufweist, gemäß Messung in einem spannungsgesteuerten Amplitudentest; bei einer Frequenz von 1 Hz und einer Temperatur von 25 °C, wobei die Fließgrenze als die 5 %ige Verringerung des Elastizitätsmoduls G' Plateauwerts, wie mit einem TA1000 Rheometer gemessen, erhältlich von TA Instruments aus New Castle, Delaware, USA, definiert ist.

7. Körperpflegeartikel nach einem der vorstehenden Ansprüche, wobei die Partikel eine Härte gemäß der Mohs Skala von weniger als 5, mehr bevorzugt weniger als 3 und noch mehr bevorzugt von weniger als 2, aufweisen.

8. Körperpflegeartikel nach einem der vorstehenden Ansprüche, wobei die Körperpflegezusammensetzung Wasser in einer Menge von mindestens 50 Gew.-% bis 98 Gew.-%, noch mehr bevorzugt von 60 Gew.-% bis 95 Gew.-%, der Körperpflegezusammensetzung aufweist.

9. Körperpflegeartikel nach einem der vorstehenden Ansprüche, wobei das Substrat eine Steifigkeit im Bereich von 5,00 g/cm bis 0,08 g/cm, vorzugsweise von 3,00 g/cm bis 0,08 g/cm, mehr bevorzugt 1,80 g/cm bis 0,10 g/cm, noch mehr bevorzugt von 0,80 g/cm bis 0,15 g/cm aufweist, wie durch ASTM D2923-06 Verfahren B ermittelt.

**10.** Körperpflegeartikel nach einem der vorstehenden Ansprüche, wobei die Partikel regelmäßig geformt sind, unregelmäßig geformt sind und eine Mischung davon, vorzugsweise ausgewählt aus kugelförmig geformten, elliptisch geformten, tetraedisch geformten, oktaederförmigen, würfelförmigen, dodekaederförmigen (pentagonale Flächen), ikosaederförmigen (dreieckige Flächen) Partikeln und Mischungen davon, mehr bevorzugt ist eine unregelmäßige Form.

**11.** Körperpflegeartikel nach einem der vorstehenden Ansprüche, wobei das Substrat ein Material umfasst und das Material wasserundurchlässig ist, vorzugsweise umfasst das Substrat eine Kunststoffschicht, mehr bevorzugt umfasst die Kunststoffschicht ein Polyolefin, mehr bevorzugt ein Polyethylen und noch mehr bevorzugt Polyethylen hoher Dichte.

**12.** Körperpflegeartikel nach einen der vorstehenden Ansprüche, der ferner eine Schutztrennschicht umfasst, die entfernbar an der Körperpflegezusammensetzung angebracht ist, vorzugsweise an einer Oberfläche der Körperpflegezusammensetzung, die nicht mit dem Substrat in Kontakt ist.

**13.** Körperpflegeartikel nach einem der vorstehenden Ansprüche, wobei die Körperpflegezusammensetzung ein Reduktionsmittel umfasst, vorzugsweise Thioglycolsäure oder ein Thioglycolatsalz, vorzugsweise in einer Menge von 0,3 Gew.-% bis 20 Gew.-%, vorzugsweise von 0,8 Gew.-% bis 15 Gew.-%, mehr bevorzugt von 1 Gew.-% bis 10 Gew.-% der Körperpflegezusammensetzung.

**14.** Verfahren zum Behandeln von Haut oder Haar, vorzugsweise Entfernen von Haar von der Haut, das folgende Schritte umfasst:

(a) Auftragen eines Körperpflegeartikels nach einem der vorstehenden Ansprüche auf die Hautoberfläche, vorzugsweise auf Säugetier- und mehr bevorzugt auf menschliche Haut,
(b) Belassen des Körperpflegeartikels in Kontakt mit der Haut für einen Zeitraum von mehr als 1 Minute, vorzugsweise 2 bis 10 Minuten, mehr bevorzugt 2 bis 8 Minuten,
(c) Entfernen des Körperpflegeartikels von der Hautoberfläche und
(d) vorzugsweise Abreiben, Abschaben, Abspülen oder Abwischen der Hautoberfläche in dem Bereich, in dem der Körperpflegeartikel aufgetragen wurde.

**15.** Körperpflegeset, vorzugsweise ein Enthaarungsset, umfassend:

(a) einen Körperpflegeartikel nach einem der Ansprüche 1 bis 13,
(b) mindestens eines von einer Vorbehandlungs-Hautpflegezusammensetzung, einer Nachbehandlungs-Hautpflegezusammensetzung und/oder eines Werkzeugs, um die Entfernung von Haar und/oder der Körperpflegezusammensetzung nach Gebrauch zu unterstützen, und
(c) Verpackung für das Set.

**Revendications**

**1.** Article de soins personnels comprenant un substrat, ledit substrat possédant une première surface, ladite surface ayant au moins une partie comprenant une composition de soins personnels disposée sur celle-ci, avec une épaisseur moyenne de composition allant de 0,01 mm à 5,00 mm, dans lequel la composition de soins personnels comprend de 1 % à 30 % en poids de particules possédant moins de 0,01 % en poids de matériau volatil ou de parfum ayant un point d'ébullition à la pression standard, inférieur à 200 °C et dans lequel le pourcentage de particules de l'épaisseur moyenne de composition va de 10 % à 100 %, calculé en divisant le diamètre moyen de particule $D_{(v,0,5)}$ tel que mesuré sur un Mastersizer 2000 de Malvern Instruments
avec l'accessoire de dispersion à sec Scirocco, par l'épaisseur moyenne de composition et en multipliant par 100, dans lequel ladite composition est aqueuse, comprenant de l'eau en une quantité supérieure à 40 %.

**2.** Article de soins personnels selon la revendication 1, dans lequel ladite au moins une partie de ladite composition de soins personnels est disposée sur la première surface du substrat avec une épaisseur moyenne de composition allant de 0,05 mm à 2,50 mm, même plus préférablement de 0,10 mm à 1,00 mm.

**3.** Article de soins personnels selon l'une quelconque revendication précédente, dans lequel lesdites particules ont une concentration allant de 1,5 % à 15,0 % en poids et plus préférablement de 2,5 % à 10,0 % en poids de la composition de soins personnels.

4.  Article de soins personnels selon l'une quelconque revendication précédente, dans lequel lesdites particules sont inertes dans la composition de soins personnels et sont de préférence solides et plus préférablement choisies parmi des coques de noix pulvérisées, une graine d'abricot séchée pulvérisée, une graine de pêche séchée pulvérisée, des céramiques pulvérulentes, du verre, du caoutchouc naturel polymère, du latex, des silicones, des cires, du talc, du phosphate tricalcique, des argiles, une zéolite, ou des mélanges de ceux-ci, plus préférablement choisies parmi du polyéthylène, du polypropylène et leurs mélanges.

5.  Article de soins personnels selon l'une quelconque revendication précédente, de préférence de 15 % à 80 %, même plus préférablement de 20 % à 50 %

6.  Article de soins personnels selon l'une quelconque revendication précédente, dans lequel ladite composition de soins personnels présente une limite d'élasticité allant de 50 Pa à 2000 Pa, de préférence de 65 Pa à 1200 Pa, plus préférablement de 80 Pa à 750 Pa lorsqu'on mesure par l'intermédiaire d'un balayage à amplitude contrôlée en contrainte ; à une fréquence de 1 Hz et à une température de 25 °C, dans lequel la limite d'élasticité est définie comme la diminution de 5 % de l'ordre de grandeur de la valeur de plateau G' du module élastique telle que mesurée sur un rhéomètre TA1000, disponible auprès de TA Instruments de New Castle, Delaware, États-Unis.

7.  Article de soins personnels selon l'une quelconque revendication précédente, dans lequel lesdites particules ont une dureté selon l'échelle de Moh inférieure à 5, plus préférablement inférieure à 3 et encore plus préférablement inférieure à 2,

8.  Article de soins personnels selon l'une quelconque revendication précédente, dans lequel ladite composition de soins personnels comprend de l'eau en une quantité de 50 % à 98 %, même plus préférablement de 60 % à 95 % en poids de la composition de soins personnels.

9.  Article de soins personnels selon l'une quelconque revendication précédente, dans lequel ledit substrat a une rigidité dans la plage allant de 5,00 g/cm à 0,08 g/cm, de préférence de 3,00 g/cm à 0,08 g/cm, plus préférablement de 1,80 g/cm à 0,10 g/cm, même plus préférablement de 0,80 g/cm à 0,15 g/cm, telle que déterminée par le procédé B de l'ASTM D2923-06.

10. Article de soins personnels selon l'une quelconque revendication précédente, dans lequel lesdites particules sont de forme régulière, irrégulière et des mélanges de ces formes, choisies de préférence parmi des particules de forme sphérique, elliptique, tétraédrique, octaédrique, cubique, dodécaédrique (à faces pentagonales), icosaédrique (à faces triangulaires) et leurs mélanges, plus préférablement de forme irrégulière.

11. Article de soins personnels selon l'une quelconque revendication précédente, dans lequel ledit substrat comprend un matériau et ledit matériau est imperméable à l'eau, de préférence le substrat comprend une feuille de plastique, plus préférablement la feuille de plastique comprend une polyoléfine, plus préférablement un polyéthylène et encore plus préférablement du polyéthylène à haute densité.

12. Article de soins personnels selon l'une quelconque revendication précédente, comprenant en outre une couche protectrice détachable fixée de façon amovible à la composition de soins personnels, de préférence sur une surface de la composition de soins personnels qui n'est pas en contact avec le substrat.

13. Article de soins personnels selon l'une quelconque revendication précédente, dans lequel la composition de soins personnels comprend un agent réducteur, de préférence de l'acide thioglycolique ou un sel thioglycolate, de préférence, présent en une quantité de 0,3 % à 20 %, de préférence de 0,8 % à 15 %, plus préférablement de 1 % à 10 % en poids de la composition de soins personnels.

14. Procédé de traitement de la peau ou des poils, de préférence d'élimination des poils de la peau, comprenant les étapes consistant à :

    (a) appliquer un article de soins personnels selon l'une quelconque revendication précédente sur la surface de la peau, de préférence une peau mammalienne, et plus préférablement la peau humaine,
    (b) laisser ledit article de soins personnels en contact avec la peau pendant une période supérieure à 1 minute, de préférence de 2 à 10 minutes, plus préférablement de 2 à 8 minutes,

(c) éliminer ledit article de soins personnels de la surface de la peau, et

(d) de préférence, frotter, racler, rincer ou essuyer la surface de la peau dans la zone sur laquelle l'article de soins personnels a été appliqué.

**15.** Trousse de soins personnels, de préférence une trousse dépilatoire, comprenant :

(a) un article de soins personnels selon l'une quelconque des revendications 1 à 13,

(b) au moins l'un parmi une composition de soins de la peau de prétraitement, une composition de soins de la peau de post-traitement et/ou un outil pour faciliter l'élimination des poils et/ou de la composition de soins personnels après utilisation, et

(c) un conditionnement pour ladite trousse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 63073910 A
- US 2006002878 A
- JP 6135826 A

- JP 11012123 A
- JP 62230711 A
- WO 0211687 A2

**Non-patent literature cited in the description**

- The Encyclopaedia of Polymers and Thickeners for Cosmetics. Department of Polymer Science, University of Southern Mississippi